# EUROPEAN PATENT APPLICATION

(11) **EP 2 147 697 A1**
(43) Date of publication of application: **27.01.2010**
(21) Application number: 08290714.8
(22) Date of filing: 21.07.2008
(51) Int. Cl.: A61N 1/32

(54) **Process and device for applying electric fields into conductive material**

(71) Applicant: Centre National de La Recherche Scientifique-CNRS, 75016 Paris (FR)
(72) Inventor: Mir, Luis Maria, 91370 Verrieres le Buisson (FR); Villemejane, Julien, 91300 Massy (FR); Le Pioufle, Bruno, 75009 Paris (FR)
(74) Representative: Collin, Jérôme

(57) **Abstract**

The present invention relates to the delivery of electric pulses any organic or inorganic conductive material and/or any biological material and/or to cells in vivo, ex vivo or in vitro, for example for the electroporation of the cells, for the electrically mediated transfer gene transfer of nucleic acids into tissue cell using a pulsed electric field and/or for the electromanipulation, in general, of the cell membrane or of the cell inside. The electric pulse applicator for the treatment of a conductive material such as biological material allowing an electric field to be applied to said conductive material in such a way as to modify it properties, comprises at least one electrode including a conductive main body and an electrically insulating coating intended to be introduced into and/or at the vicinity of the conductive material to be treated, and a pulse generator sending pulses to the electrodes having a slope (dE/dt) comprised between 10¹⁴ and 10¹⁸ V/m/s.

## Description

### BACKGROUND OF THE INVENTION

The present invention is related to the delivery of electric pulses any organic or inorganic conductive material and/or any biological material and/or to cells in vivo, ex vivo or in vitro, for example for the electroporation of the cells, for the electrically mediated transfer gene transfer of nucleic acids into tissue cell using a pulsed electric field and/or for the electromanipulation, in general, of the cell membrane or of the cell inside.

Electrically mediated gene transfer, also termed DNA electrotransfer or electrogenetherapy, uses various single or multiple-electrode designs such as arrays of two or more electrodes that typically are designed as needle electrodes for insertion into said tissue, said electrode being connected to a pulse generator. The method has been shown to be effective to electrotransfer plasmid DNA to various tissues: muscles, liver, skin, tumors, mouse testis, etc...

The mechanisms by which electric pulses mediate DNA transfer into target cells are not well understood. Nevertheless, there is a common agreement that for an improved DNA transfer into tissues, cells in that tissue must be permeabilized. Such a permeabilization can be achieved using simple runs of short square wave electric pulses (in the range of 100 [mu]s). This kind of pulses has been widely used for the local delivery of non-permeant anticancer drugs (like bleomycin or cisplatin) in a treatment termed 'antitumor electrochemotherapy'. Indeed, the delivery to tumors of e.g. 8 pulses of 1300 V/cm and 100 [mu]s either in vitro or in vivo is sufficient to induce transient rearrangements of the cell membrane that allow non-permeant anticancer molecules like bleomycin to enter the cell by diffusion and to fully exert their cytotoxic activity.

These short permeabilizing electric pulses have also been shown to increase the transfer of plasmid DNA into several tissues. However, another type of square-wave electric pulses was applied to muscles, tumors, liver and some other tissues, and was found to be more effective for DNA electrotransfer. These pulses usually are of lower voltage but much longer duration (in the range of tens of milliseconds). It is assumed that this type of pulses mediate DNA transfer into the cells by inducing two distinct effects that include cell permeabilization (like the short pulses) and DNA electrophoretic migration during the delivery of the electric field.

Efficient electrotransfer into cells has been described in WO-A-99/01158 and in WO-A-98/43702 notably.

For the years 1960-1970, *in vitro* studies showed that pulsed electric fields (PEF) delivery on living cells induce a reversible or irreversible breakdown of the cell membranes, called electropermeabilization (Coster HG (1965). A quantitative analysis of the voltage-current relationships of fixed charge membranes and the associated property of "punch-through". Biophys J 5: 669-686.; Sale AJ and Hamilton WA (1968). Effects of high electric fields on micro-organisms. 3. Lysis of erythrocytes and protoplasts. Biochem Biophys Acta 163: 37-73.; Neumann E and Rosenheck K (1972). Permeability changes induced by electric pulses in vesicular membranes. J Membr Biol 10: 279-290.; Crowley JM (1973). Electrical breakdown of bimolecular lipid membranes as an electromechanical instability. Biophys J 13: 711-724.; Zimmermann U, Pilwat G, Rieman F (1974). Dielectric breakdown of cell membranes. Biophys J 14: 881-899.). In the range of 100 µs to 100 ms and 25 to 1500 V/cm, PEF are used to allow genes or molecules entering the cells (Jaroszeski MJ, Gilbert R, Nicolau C, Heller R (2000). Delivery of genes in vivo using pulsed electric fields. In Jaroszeski MJ, Heller R, Gilbert R, editors. Electrochemotherapy, electrogenetherapy and transdermal drug delivery: electrically mediated delivery of molecules to cells. Totowa, New Jersey: Human press: 173-186). Associated with some classical chemotherapeutic drugs (bleomycin or cisplatin), it can vectorize drugs in cancer tissues without damaging those around (Belehradek J, Orlowski S, Ramirez LH, Pron G, Poddevin B, Mir LM (1994). Electropermeabilization of cells in tissues assessed by the qualitative and quantitative electroloading of blemomycin. Biochim Biophys Acta 640: 169-178.; L.M. Mir, J. Gehl, G. Sersa, C. Collins, JR Garbay, V. Billard, P. Geertsen, Z. Rudolf, G. O'Sullivan, M. Marty (2006). Standard Operating Procedures of the Electrochemotherapy: Instructions for the use of bleomycin or cisplatin administered either systemically or locally and electric pulses delivered by the Cliniporator™ by means of invasive or non-invasive electrodes" Eur. J. of Cancer Supplements, special issue "Electrochemotherapy", 4, 14-25.; M. Marty, G. Sersa, JR Garbay, J.Gehl, C. Collins, M. Snoj, V. Billard, P. Geertsen, J. Larkin, D. Miklavcic, I Pavlovic, S. Paulin-Kosir, M. Cemazar, N. Morsli, D. Soden, Z. Rudolf, C. Robert, G. O'Sullivan and L.M. Mir. (2006) Electrochemotherapy - a simple, highly effective and safe treatment of cutaneous and subcutaneous metastases: results of ESOPE (European Standard Operating Procedures for Electrochemotherapy) study. Eur. J. of Cancer Supplements, special issue "Electrochemotherapy", 4, 3-13.). This technique is the electrochemotherapy. Another technique, called gene electrotransfer and based on the same physical method, is used to internalize DNA plasmids in cells without causing irreversible damages on plasma membranes (Neumann E, Schaefer-Rideer M, Wang Y, Hofschneider PH (1982). Gene transfer into mouse lyoma cells by electroporation in high electric fields. EMBO J 1: 841-845. ; Mir LM, Bureau MF, Gehl J, Rangara R, Rouy D, Caillaud JM, Delaere P, Branellec D, Schwartz B, Scherman D (1999). High-efficiency gene transfer into skeletal muscle mediated by electric pulses. Proc Natl Acad Sci USA 96: 4262-4267.).

A new kind of PEF, nanosecond pulsed electric fields (nsPEF) is actually under study. nsPEF are ultra-short pulses (10 to 300 ns) with higher electric field strength (10 to 150 kV/cm) that do not increase the temperature of the exposed cells (Schoenbach KH, Beebe SJ, Buescher ES (2001). Intracellular effect of ultrashort electrical pulses. Bioelectromagnetics 22: 440-448). First studies showed that nsPEF induced permeabilization of intracellular membranes (granules, vesicles, mitochondria, nucleus...) but not of plasma membrane (Schoenbach KH, Beebe SJ, Buescher ES (2001). Intracellular effect of ultrashort electrical pulses. Bioelectromagnetics 22: 440-448; Beebe SJ, White J, Blackmore PF, Deng Y, Somers K, Schoenbach KH (2003). Diverse effects of nanosecond pulsed electric fields on cells and tissues. DNA Cell Biol 22: 785-796.). However Chen N, Schoenbach KH, Kolb JF, Swanson RJ, Garner AL, Yang J, Joshi RP, Beebe SJ (2004). Leukemic cell intracellular responses to nanosecond electric fields. Biochem Biophys Res Commun 317: 421-427. indicate a possible poration of the membrane during the application of multiple nsPEF or long nanopulse.

Several studies have been published on nsPEF effects on animal cells, in particular on apoptosis. These studies report an induction of apoptotic markers like caspase activation, phosphatidylserine externalization and cytochrome c release into the cytoplasm (Vernier PT, Sun Y, Marcu L, Craft CM, Gundersen MA (2004). Nanoelectropulse-induced phosphatidylserine translocation. Biophys J 86: 4040-4048.; Hall EH, Schoenbach KH, Beebe SJ (2005). Nanosecond pulsed electric fields (nsPEF) induce direct electric field effects and biological effects on human colon carcinoma cells. DNA Cell Biol 24: 283-291.). DNA damages have been found (Stacey M, Stickley J, Fox P, Statler V, Schoenbach KH, Beebe SJ, Buescher S (2003). Differential effects in cells exposed to ultra-short, high intensity electric fields: cell survival, DNA damage, and cell cycle analysis. Mutat Res 542: 65-75.; Chen N, Schoenbach KH, Kolb JF, Swanson RJ, Garner AL, Yang J, Joshi RP, Beebe SJ (2004). Leukemic cell intracellular responses to nanosecond electric fields. Biochem Biophys Res Commun 317: 421-427) as well as a cellular specificity of nsPEF effects on cells. Stacey *et al.* "Stacey M, Stickley J, Fox P, Statler V, Schoenbach KH, Beebe SJ, Buescher S (2003). Differential effects in cells exposed to ultra-short, high intensity electric fields: cell survival, DNA damage, and cell cycle analysis. Mutat Res 542: 65-75" demonstrated a decrease in viability and an increase in DNA damage in suspension cells compared to adherent cells.

nsPEF also have been shown to induce (i) a release of intracellular calcium from the endoplasmic reticulum in cells under conditions maintaining plasma membrane integrity (Stacey M, Stickley J, Fox P, Statler V, Schoenbach KH, Beebe SJ, Buescher S (2003). Differential effects in cells exposed to ultra-short, high intensity electric fields: cell survival, DNA damage, and cell cycle analysis. Mutat Res 542: 65-75.; Vernier PT, Sun Y, Marcu L, Salemi S, Craft CM, Gundersen MA (2003). Calcium bursts induced by nanosecond electric pulses. Biochem Biophys Res Commun 310: 286-295.; White JA, Blackmore PF, Schoenbach KH, Beebe SJ (2004). Stimulation of capacitative calcium entry in HL-60 cells by nanosecond pulsed electric fields. J Biol Chem 279: 22964-22972.); and (ii) an enhancement of gene transfection efficiency. Within these papers, one experiment showed that the application of 1 nsPEF (10 ns, 150 kV/cm) 30 min after the GFP gene electrotransfer into cells in suspension allows an increase of 3-fold of the GFP expression compared to electrotransfer only (Beebe SJ, Fox PM, Rec LJ, Willis LK, Schoenbach KH (2003). Nanosecond, high-intensity pulsed electric fields induce apoptosis in human cells. FASEB J 17: 1493-1495., White JA, Blackmore PF, Schoenbach KH, Beebe SJ (2004). Stimulation of capacitative calcium entry in HL-60 cells by nanosecond pulsed electric fields. J Biol Chem 279: 22964-22972.). As the electrogenetransfer, like the other approaches for non viral gene therapy, is considered less efficient than the viral approaches for gen therapy, an increase of 3-fold or more of the GFP reporter gene expression is very important for the development of this non-viral gene therapy approach, which is considered, in general, safer and easier than the viral approaches.

Moreover, electroporation has been applied to delivering molecules to subsurface tissues using various single or multiple-electrode designs such as arrays of two or more electrodes that typically are designed as needle electrodes for insertion into said tissue, said electrode being connected to a pulse generator. Generally, such arrays define a treatment zone lying between the needle electrodes of the array. Such treatment zones therefore comprise a three dimensional volume of tissue wherein cells within the treatment zone are exposed to an electric field of an intensity sufficient to cause temporary or reversible poration, or even sometimes irreversible poration, of the cell membranes to those cells lying within and or near the three dimensional volume.

The US patent US 5,674,267 discloses such a process and an electric pulse applicator for the treatment of biological tissue applying an electric field to the cells of biological tissue to modify the properties of their membranes.

Current practices for electroporating cells in tissue include use of significant voltages in order to impart through the three dimensional treatment zone a relatively uniform electric field. By "relatively uniform" is meant that electric lines of force coincident with application of an electric pulse sufficient to cause poration is imparted across the cells somewhat evenly throughout the three dimensional treatment zone volume.

Besides the invasive aspect of a device with multiple needles, typical electroporation techniques, as stated above, result in variability in electroporation of cells within a treatment zone. This is a drawback to medical use of electroporation in that dispersion of treatment molecules of the injected bolus into surrounding tissue results in loss of control as to the amount of such treatment molecule that is ultimately transfected into cells within the treatment zone by the electroporation event.

Moreover, the use of metallic electrodes on contact of the skin or of the biological tissues may cause burns which are visible on the skin and which can be painful for a patient. These burns are probably of electrochemical kind. Indeed, the oxidizable metal of electrodes and the molecule of H2O and NaCl present in the surrounding of electrodes and on contact of said electrodes create various reactive species when the pulses are delivered. To avoid, or to reduce these burns, it is necessary to use biocompatible materials, for example specific metals or alloys, to elaborate the electrodes. This constraint may preclude the use of materials with optimal electrical properties (conductivity, permittivity) that may contain heavy metals, toxic ions, or, in general, non biocompatible substances. The electrochemical burns may affect normal cells reducing the efficacy of the electrogenetransfer or reducing the volumes treated by electrochemotherapy (as the electric pulses by themselves does not kill the cells in this application, and the bleomycin is killing almost exclusively the malignant tumor cells and sparing the non-dividing normal cells). Moreover, the ultrashort nanopulses seem to be unable to provoke the contraction of the muscles located in the contact or close to the electrodes, which can add comfort to the patient with respect to the treatment by electrochemotherapy using classical 100 µs-long pulses.

Consequently, there is a need to enhance the electrically mediated transfer gene transfer of nucleic acids into tissue cell using a pulsed electric field and/or to procure an electroporation process that do not damage healthy cells.

### BRIEF DESCRIPTION OF THE INVENTION

The above-mentioned need is addressed by the embodiments described herein in the following description.

In one embodiment, an electric pulse applicator for the treatment of a conductive material such as biological material allowing an electric field to be applied to said conductive material in such a way as to modify it properties is provided. Said pulse applicator comprises
at least one electrode including a conductive main body and an electrically insulating coating intended to be introduced into and/or at the vicinity of the conductive material to be treated,
a pulse generator sending pulses to the electrodes having a slope (dE/dt) comprised between 10¹⁴ and 10¹⁸ V/m/s

The pulses have an amplitude of about 10 to 200 kV/cm and a pulse length of one or several hundreds of picoseconds to one or several tens or hundreds of nanoseconds.

Preferably, each pulse has a duration lower than 1 microsecond.

Advantageously, each pulse has a length comprised between 1 and 10 nanoseconds.

Moreover, said electrically insulating coating is an insulating inorganic film such as an insulating polymer film or an insulating elastomer film.

Alternatively, said electrically insulating coating is an insulating mineral film obtained for example from the following list of minerals : glass, oxide, nitride, etc...

Alternatively, said electrically insulating coating is an insulating organic film such as an insulating cellulose film, an insulating lipidic film, or similar.

Said electrically insulating coating is a PDMS (Polydimethylsiloxane) film.

Moreover, said electrically insulating coating presents a thickness of about or less of 0.5 mm.

In another embodiment, an electrode intended to be introduced into and/or at the vicinity of a conductive material to be treated, for an electric pulse applicator for the treatment of conductive material, said electric pulse applicator comprising a pulse generator sending pulses to the electrodes having a slope (dE/dt) comprised between 10¹⁴ and 10¹⁸ V/m/s, wherein said electrode includes a conductive main body and an electrically insulating coating.

Moreover, said electrically insulating coating is an insulating inorganic film such as an insulating polymer film or an insulating elastomer film.

Alternatively, said electrically insulating coating is an insulating mineral film obtained for example from the following list of minerals : glass, oxide, nitride, etc...

Alternatively, said electrically insulating coating is an insulating organic film such as an insulating cellulose film, an insulating lipidic film, or similar.

said electrically insulating coating is a PDMS (Polydimethylsiloxane) film.

Moreover, said electrically insulating coating presents a thickness of about or less of 0.5 mm.

In yet another embodiment, a method for applying an electric field into a conductive material in such a way as to modify it properties is provided. Said method comprises at least the following step of:
- positioning at least one electrode comprising a conductive main body and an electrically insulating coating into and/or at the vicinity of the conductive material to be treated,
- sending pulses to the electrode having a slope (dE/dt) comprised between 10¹⁴ and 10¹⁸ V/m/s

The pulses have an amplitude of about 10 to 200 kV/cm and a pulse length of one or several hundreds of picoseconds to one or several tens or hundreds of nanoseconds.

Preferably, each pulse has a length lower than 1 microsecond.

Advantageously, each pulse has a length comprised between 1 and 10 nanoseconds.

### BRIEF DESCRIPTION OF THE DRAWINGS

Fig. 1 illustrates a schematic representation of an electric pulse applicator according to the invention,

Fig 2 illustrates a schematic representation of the section of a planar electrode of the electric pulse applicator according to the invention (or of a section of a no-planar electrode).

Fig 3 illustrate a representation of an experimental electric pulse applicator according to the invention,

Fig 4 illustrates a detailed of an experimental electric pulse applicator according to the invention,

Fig 5 to 15 illustrates the results of different experiments in vivo and in vitro with prior art electric pulse applicator and with the electric pulse applicator according to the invention.

### DETAILED DESCRIPTION OF THE INVENTION

In the following detailed description, reference is made to the accompanying drawings that form a part hereof, and in which is shown by way of illustration specific embodiments, which may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken in a limiting sense.

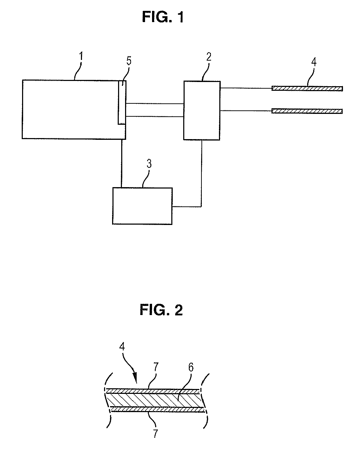

Referring to figure 1, the device for applying an electric field into biological material comprises a pulse generator 1, a selector switch 2, a control unit 3 and at least one electrode 4. Pulse generator 1 comprises a high voltage power supply 5 which is connected to the mains supply.

The device according to the invention is intended to apply a variable electric field to cells and/or any biological material and/or any organic or inorganic conductive material located between a pair of electrodes 4.

Each electrode 4 can be connected either to the positive or negative pole of the high voltage power supply 5.

Moreover, each electrode includes a metallic main body 6, made in aluminium, copper, etc..., or any conductive material, coated by an electrically insulating material 7. Said electrically insulating coating 7 can be an insulating inorganic, organic or mineral film such as a PDMS (Polydimethylsiloxane) film, an insulating glass, oxide, nitride, etc...film, an insulating cellulose, lipidic, etc...film, an insulating elastomer or polymer film, etc... for example. The thickness of said insulating film can be about or less than 0.5 mm for example. Note that the thickness of the electrically insulating layer can be greater for specific industrial application without departing from the scope of the invention.

Referring to figure 1 to 4, each electrode 4 has a rectangular planar shape as a chip, the biological material being placed between two parallel electrodes 4.

Note that the electrode 4 can have any shape, as for example a disc shape, without departing of the scope of the invention.

Moreover, each electrode 4 can consist in a needle coated by an insulating material and comprising a base, a head and a connector as disclosed in the US patent US 5,674,267, or in any other kind of electrode already known by the man skilled in the art.

Control unit 12 controls the high tension power supply 13 and changeover switch 11 according to the instructions it receives from an operator or via a program.

The device according to the invention is thus able to apply previously determined pulse cycles between electrodes 4. The pulses applied to each electrode 4 are rectangular-shape pulses, or trapezoidal, or triangular, or sinusoidal, or similar or have a shape which spectrum contains at least the spectrum of above mentioned signals, having an amplitude of about 100 V/cm to 200 kV/cm and a pulse length lower than 1 microsecond, and preferably comprised between 0.1 and 10 nanoseconds, and preferably of less than a nanosecond or a few nanoseconds, with a slope (dE/dt) of the raising front comprised between 10¹⁴ and 10¹⁸ V/m/s.

In these conditions of pulse, the electrically insulating coating 7 of electrodes 4 loose its insulating properties allowing the generation of a "nanopulsed" electrical field.

The whole of the electrode 4 is coated by an electrically insulating film, and the electric field that is generated in the biological object (cells, tissues, organs) or in any conductive non-biological object placed between the coated electrodes also pass through the insulating film. Of course in the present invention, the electrodes 4 can be completely coated, or they can be partially uncoated in the parts that are far from the biological or non biological object submitted to the electric pulses, or in the parts where two adjacent electrodes are the most apart, for example to facilitate the electrical connections with the pulse generator

It could be noted that the amplitude and the length of the pulse will be adapted by the operator in function of the use of the device and the kind of biological material: electrically mediated gene transfer of nucleic acids into tissue cell and/or electroporation and/or destruction of cells by irreversible electroporation, and/or any cell electromanipulation made feasible by the use of the nanopulses.

The device according to the invention can notably be used for a tumor treatment by electrochemotherapy and/or electrotherapy and/or genetherapy.

The tumor treatment by electrochemotherapy consists then in the following steps:
- injecting a non-permeant anticancer drugs (like bleomycin or cisplatin) either systemically in the body or locally in or at the vicinity of the tumor,
- introducing at least one electrode (such a needle) into the tumor (or its vicinity) beforehand detected,
- generating at least one pulsed electrical field having an amplitude of about 100 V/cm to 200 kV/cm and a pulse length of less than a nanosecond or of a few nanoseconds.

The tumor treatment by electrotherapy consists then in the following steps:
- introducing at least one electrode (such a needle) into the tumor beforehand detected,
- generating at least one pulsed electrical field having an amplitude of about 10 to 200 kV/cm and a pulse length of a few nanoseconds to destroy said tumor.

Note that the use of the device according to the invention in the tumor treatment by electrochemotherapy gives the advantage to destroy the tumor without burning surrounding healthy cells and without deposition of metallic parts in the surrounding cells.

The improvement of gene transfer to cells in vitro, to tissues ex vivo and in vivo to tumors or to any other tissue, like the skeletal muscle consists then in the following steps:
- injection of a material including a specific gene or a short nucleic acid in the tissue encompassed by the electrodes
- introducing the material into the cells by classical electrogenetransfer procedures or by procedures in which electroporation is achieved by electric pulses using the electrodes according to the present invention.
- generating, before or after the electrogenetransfer at least one supplementary pulsed electrical field having an amplitude of about 1 to 200 kV/cm and a pulse length of one or several hundreds of picoseconds to one or several tens or hundreds of nanoseconds to improve the efficiency of the electrogenetransfer.

Moreover, the device according to the invention allows to free electrodes from biocompatibility constraints. In such a way, the main body of electrodes 4 can be obtained in any desired conductive material. This material can thus have different electrical properties (conductivity, permittivity) optimal for the tissue and for the desired procedure or treatment, without being limited to a choice among biocompatible authorized materials

### Example 1

We explored whether nanopulses (nsPEF, electric pulses of duration of ten nanoseconds and of very high electric field strength with a device of prior art comprising electrodes without an electrically insulating coating) could affect the expression of the reporter gene luciferase, improving the overall efficiency of the non-viral, electric pulses mediated, gene transfer. We analyzed several parameters like number of nsPEF, nsPEF repetition frequency, delay between the DNA electrotransfer and nsPEF delivery, amplitude and amount of DNA. In a second time, we wanted to determine involved mechanisms by studying nsPEF effects (i) on release of intracellular calcium and (ii) on nuclear pore transport and (iii) on plasmid transcription.
We showed an increase of 3-fold of the luciferase gene expression with the application of just 1 nsPEF of 60 kV/cm, 60 minutes after plasmid electrotransfer, in an electroporation cuvette with 1 mm of distance between the electrodes. However, more than one pulse can also applied, as no loss of viability is associated to the exposure to these nanopulses. The time between DNA electrotransfer and nanopulse(s) delivery (between 15 and 180 minutes) is not relevant which seems to indicate that the effect of the nanopulses is not at the level of the DNA internalization. Mechanisms are under analysis, as cell manipulation by means of electric nanopulses delivery might be an efficient way to increase the overall efficacy of gene electrotransfer.

### INTRODUCTION

The objective of this study was to explore whether nsPEF actually affect the efficiency of plasmids electrotransfer *in vitro,* analyzing several parameters like number of nsPEF, nsPEF repetition frequency, delay between the DNA electrotransfer and nsPEF delivery, amplitude and amount of DNA. In a second time, we wanted to determine involved mechanisms by studying nsPEF effects (i) on release of intracellular calcium and (ii) on nuclear pore transport and (iii) on plasmid transcription.

### MATERIALS AND METHODS

### nsPEF exposure system

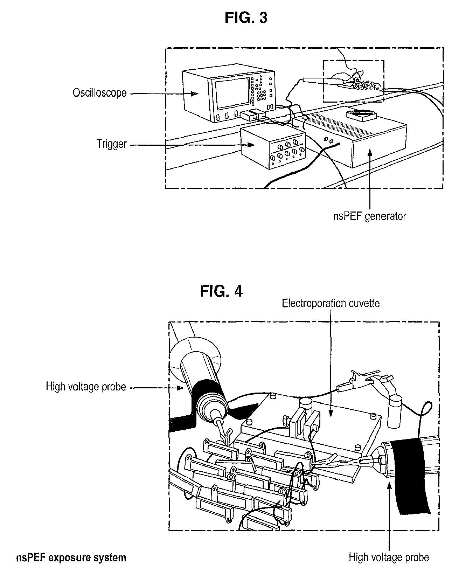

nsPEF were delivered with a high voltage generator FPG 10-30MS (FID Technology, Russia). It can deliver electric pulses from 2.5 kV to 10 kV per output in impedance of 1000 ohms and it has 4 similar ones. Pulses last 10 ns and have transition time of 3 ns. An external trigger from TTY is used to set off the nsPEF generator (Figure 3). Applied signal was visualized with an oscilloscope LeCroy WavePro 7000 and two high voltage probes Tektronix P6015A (1000X, 40 kV max). Cell suspensions were exposed to nsPEF in two types of electroporation cuvettes: 1 or 2 mm between the electrodes (Figure 4).

### Cell culture

DC-3F cells (Chinese hamster fibroblast lung cells) and LPB cells (mouse fibrosarcoma) were grown in the complete medium: Minimum Essential Medium (Invitrogen, Cergy-Pontoise, France) supplemented with 10% fetal bovine serum (Invitrogen), 500 U/ml penicillin, 500 µg/ml streptomycin (Invitrogen) defined as complete medium. Cultures were maintained in a humidified atmosphere with 5% CO₂ at 37°C. Cells were routinely passed every two days.

### Plasmid DNA

Plasmid pCMV-Luc (Clontech, Montigny-les-Bretonneux, France) was prepared using the Endotoxin-free Plasmid DNA (Macherey-Nagel, Hoerdt, France) according to manufacturer's protocol.

### DNA electrotransfer

Cells were harvested by trypsin and cell suspension was placed into electroporation cuvettes in low conductivity medium (250 mM sucrose, 10 mM Tris, 1 mM MgCl₂, pH=7) (1×10⁶ cells per electroporation cuvette). Cells were exposed to 2 electropermeabilizing pulses (1250 V/cm, 100 µs, 1 Hz) delivered by a Cliniporator (IGEA, Carpi, Italy) in the presence of DNA coding for the luciferase. After these pulses, cells were incubated for 30 to 180 min either at room temperature or at 37°C under 5% CO₂.

### Nanosecond pulsed electric fields (nsPEF) delivery

Cells were exposed to nsPEF in electroporation cuvettes (Molecular BioProducts, VWR, France) which had a gap between the electrodes of 1 or 2 mm. After the nsPEF delivery, cells were removed from the electroporation cuvette and cultured in the complete medium for 24 or 48 hours at 37°C under 5% CO₂. The luciferase activity and the total protein concentration were measured as described below.

### Exposure to trans-cyclohexane-1,2-diol (TCHD)

After the DNA electrotransfer, cells were incubated with 40 mM TCHD (Sigma-Aldrich, L'lsle-d'Abeau-Chesne, France) for 1 hour at room temperature and then exposed or not to 20 nsPEF of 100 kV/cm with a repetition frequency of 1 or 10 Hz. After the exposure to the nsPEF, the non-conductive medium was removed and replaced by complete medium. Cells were cultured for 24 hours at 37°C under 5% CO₂ and the luciferase activity and total proteins concentration were measured.

### Determination of luciferase expression

The luciferase expression was determined using the Luciferase Assay System (Promega, Charbonnieres, France). The cells were harvested and centrifuged at 1000 rpm for 10 minutes. The pellet was then resuspended in 200 µl lysis buffer (Promega), centrifuged at 11000 rpm for 1 minute and the supernatant was collected. To correct for the amount of cells per cuvette, the protein concentration in cell supernatant was determined with the Micro BCA^{™} Protein Assay Kit (Pierce, Perbio Science France SAS, Brebieres, France).

The measurement of luciferase activity was carried out by adding 20 µl of cell supernatant to 100 µl luciferase buffer, using a Lumat LB 9507 luminometer (Berthold France SA, Thoiry, France) by integration of the light produced during 10 seconds. The results were collected from the luminometer in relative light units (RLU). Calibration with several concentrations of purified firefly luciferase protein (Promega) was performed to convert RLU in pg of luciferase using the following linear conversion: Log (pg) = 1.24 log (RLU) - 4.83. The final results were expressed as pg of luciferase per µg of total proteins.

### Fluorometric analysis of internal calcium response

The fluorescent indicator Calcium Green-1-AM (Invitrogen, France) was used with a spectrofluorometer (SFM 25, Kontron Instruments). Cells were incubated with Calcium Green-1-AM (1 µM) in growth medium for 45 min at 37°C in the culture dishes. The cells were then washed in PBS, trypsinized and resuspended in the non-conductive sucrose buffer. Cells were first placed in the fluorometer cuvette to determine a base-line reading, and then transferred from the fluorometer cuvette to the electroporation cuvette. Cells were treated with nsPEF and then immediately transferred back from the electroporation cuvette to the fluorometer cuvette (the spectrofluorometer being located in close proximity to the pulse generator, the procedure took between 5 and 10 seconds), and the fluorescence measurements were realized.

### Data analysis

Results are reported as the ratio of the pg of luciferase per mg of total proteins in the exposed (electrotransfer plus nsPEF) to the pg of luciferase per mg of total proteins in the controls (electrotransfer only).
All data presented are mean values ± SD from three independent experiments. Statistical analysis was performed using Student's t-test. A p value lower than 0.05 was considered statistically significant.

### RESULTS

### The number of nsPEF

45 min after the luciferase gene electrotransfer 1, 2, 5, 10 or 20 nsPEF of 10 ns and 60 kV/cm were applied to DC-3F cells. No decrease of cell survival associated to nsPEF exposure was found. The results concerning the luciferase activity readings differed whether the cuvette had a gap of 1 or 2 mm between the electrodes.

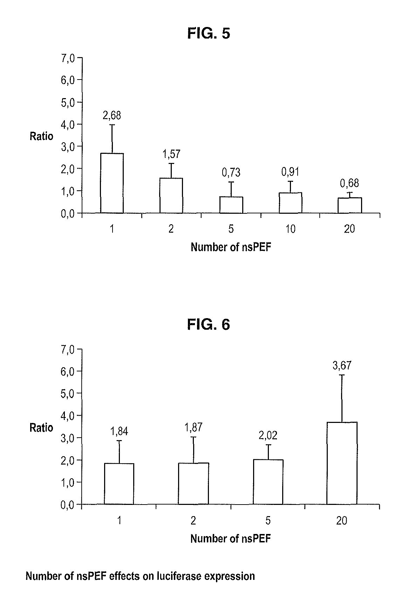

In the cuvettes of 1 mm, 1 nsPEF was sufficient to increase about 3-times the luciferase expression compared to the electrotransfer only (Figure 5). In contrast, in the cuvettes of 2 mm, 20 nsPEF were required to obtain the same increase of the luciferase expression (Figure 6).

### The repetition frequency of nsPEF

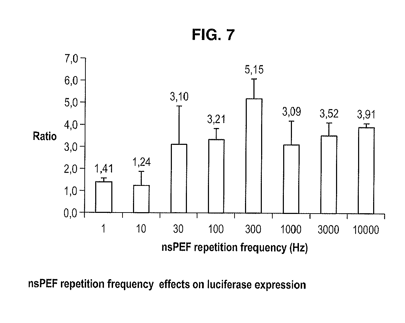

DC-3F cells were exposed to 20 nsPEF of 45 kV/cm, 45 min after DNA electrotransfer in electroporation cuvettes with 2 mm between the electrodes. Results obtained show that effects are independent of the nsPEF repetition frequency (30 to 10000 Hz) (Figure 7).

### The delay between the gene electrotransfer and the application of nsPEF

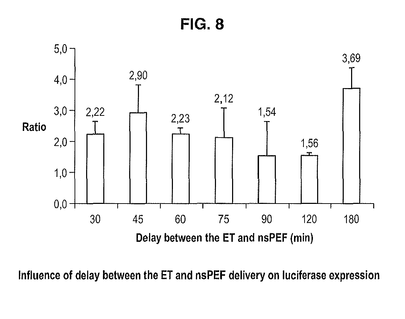

DC-3F cells were exposed to 20 nsPEF, 1 Hz, of 60 kV/cm in electroporation cuvette with 2 mm between the electrodes. The time between DNA electrotransfer and nsPEF delivery (between 15 and 180 min) seems to be not relevant which seems to indicate that the effect of the nsPEF is not at a level of the DNA uptake (Figure 8).

### The amount of DNA in cuvette

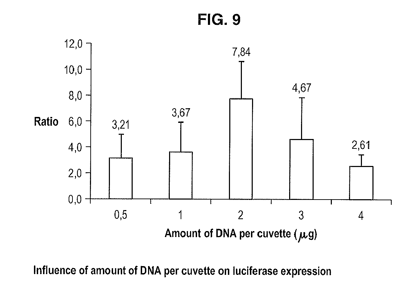

60 minutes after DNA electrotransfer, DC-3F cells were exposed to 1 nsPEF of 45 kV/cm in cuvette of 1 mm between the electrodes. Whatever is the amount of DNA used (0.5, 1, 2, 3 or 4 µg per cuvette), nsPEF increased luciferase expression compared to the controls. Nevertheless, a most important increase was observed for 2 µg of DNA (Figure 9).

### Mechanism: do nsPEF affect nuclear pores?

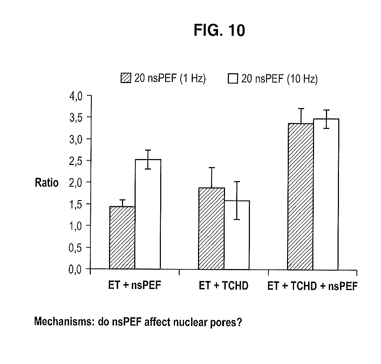

30 min after DNA electrotransfer, LPB cells were exposed to TCHD (40 mM) ± nsPEF (100 kV/cm, 20 nsPEF with a repetition frequency of 1 or 10 Hz). The exposure to TCHD resulted in a two-fold increase in gene expression, as foreseen according to Vandenbroucke *et al.* (2007). Whatever the nsPEF repetition frequency, the cells exposed to the nsPEF alone showed again an increase of gene expression (of about 2-fold the rate in control in these experiments) which was similar to increase observed in cells exposed to TCHD. When cells were exposed to nsPEF + TCHD, additive effects were observed (Figure 10).

### Fluorometric analysis of internal calcium release

In its acetoxymethylester form, Calcium Green-1-AM is nonfluorescent and membrane permeable. Inside the cell, esterases cleave the acetoxymethylester.
DC-3F cells were exposed to 1 or 20 nsPEF (1 Hz) in cuvette of 2 mm or 1 mm. Fluorescence was measured before and after the pulses.

### DISCUSSION

In this report, we confirm that cell manipulation by means of electric nsPEF delivery may efficiently increase the overall efficiency of gene electrotransfer. An increase of three fold in the production of the reporter gene was achieved. However, it is worth mentioning that this increase in luciferase activity was achieved under precise conditions. Indeed, while some parameters does not seem crucial (time between DNA electrotransfer and nsPEF delivery, or the repetition frequency when several nsPEF were delivered), other parameters are very important. In particular we found a correlation between the number of nsPEF necessary to achieve an effect and the distance between the electrodes. With a gap of 1 mm, 1 nsPEF is sufficient to stimulate luciferase production after luciferase gene electrotransfer. With a gap of 2 mm, 20 nsPEF were required. We are presently analyzing this surprising observation. Because of the very short duration of the nsPEF, we are analyzing the shape of the pulse generated by the nanopulse generator used in these studies. The number of pulses required could relate to the distribution of the electric field in the cuvette during the pulse. It must be highlighted that the nsPEF pulses were not rectangular nor even trapezoidal, but rather triangular which means that the sterady state is not reached during the pulse. Even though we have not yet concluded the investigations of the influence of the pulse shape on the number of nsPEF required to reach the effect reported here, the message is that the exposure conditions are crucial to achieve the reported increase in the efficiency of DNA electrotransfer.
We have started to analyze the mechanisms of the increase in reporter gene activity here reported. Because there is no influence of the delay between the DNA electrotransfer and the nsPEF delivery, the reported increase should not result of an increased uptake. Indeed, we know that after 1 or 2 pulses of 100 µs and 1250 V/cm, cells remain open for a few minutes, but not for one hour.
TCHD - known to enhance the transfection efficiency making the nucleus permeable for plasmid DNA or high molecular weight molecules (Vandenbroucke *et al*., 2007) - additivity - not affect the increase in efficacy caused by the nsPEF which suggests that the effect of the nsPEF is not at the level of the DNA transport from cytosol to nucleus
Ca++ peaks after pulses delivery were observed, as already described in the literature.

### Example 2

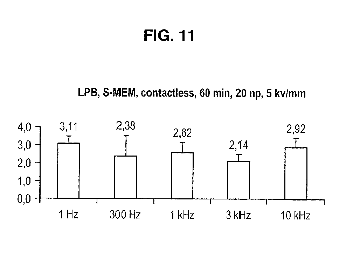

The same method had been applied with a device according to the invention including a pair of electrodes 4 comprising an electrically insulating coating as described above and best results had been obtained as illustrated in figure 11. Other methods are similar to those of example 1.

### Example 3

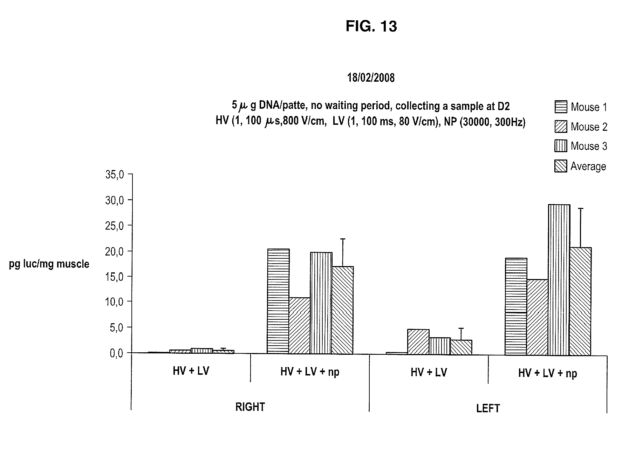

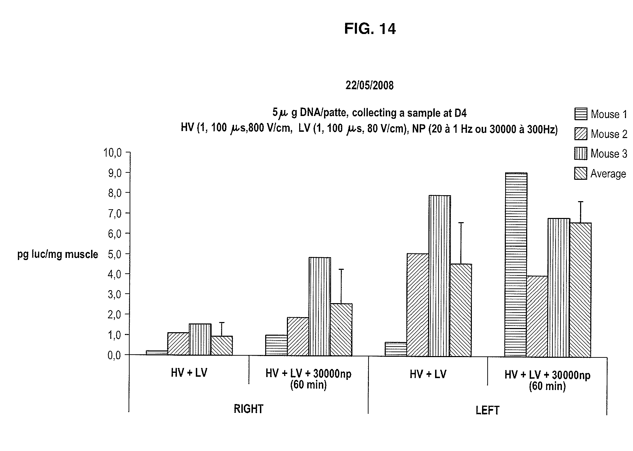

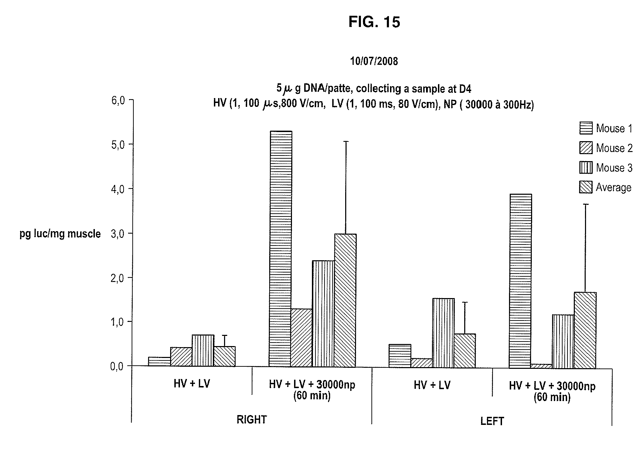

We explored whether nanopulses (nsPEF, electric pulses of a duration of a few tens of nanoseconds and of very high electric field strength with a device according to the invention comprising electrodes including an electrically insulating coating) could affect the expression of the reporter gene luciferase electrotransferred into the *tibialis cranialis* skeletal muscle of mice legs. The results are reported in figures 13 to 15. Methods are those described in example 1 (in particular for the nanopulse generator) or methods classically found in the literature for experiments concerning DNA electrotransfer in skeletal muscle in mice.

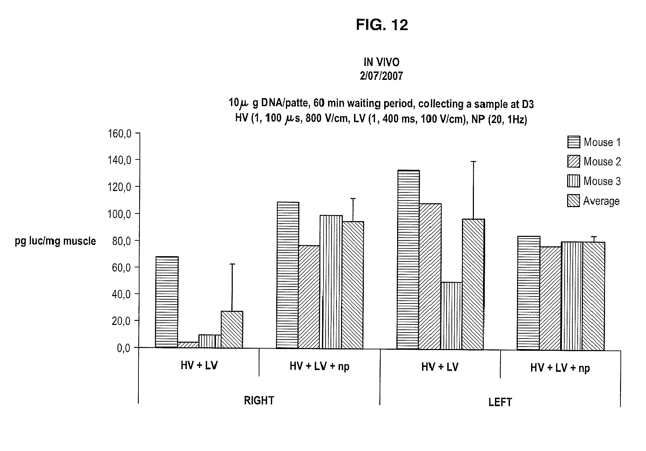

It appears that the application of "nanopulsed" enhance the expression of the reporter gene luciferase under certain conditions (Figures 12 to 15). The right protocol for a determined biological material and for a determined application will be easily determined with a routine by the man skilled man in the art.

This written description uses examples to disclose the invention, including the best mode, and also to enable any person skilled in the art to make and use the invention. The scope of the subject matter described herein is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An electric pulse applicator for the treatment of a conductive material such as biological material allowing an electric field to be applied to said conductive material in such a way as to modify it properties, comprising
at least one electrode including a conductive main body and an electrically insulating coating intended to be introduced into and/or at the vicinity of the conductive material to be treated,
a pulse generator sending pulses to the electrodes having a slope (dE/dt) comprised between 10¹⁴ and ₁₀¹⁸ V/m/s.

2. Electric pulse applicator according to claim 1 wherein the pulses have an amplitude of about 10 to 200 kV/cm and a pulse length of one or several hundreds of picoseconds to one or several tens or hundreds of nanoseconds.

3. Electric pulse applicator according to claim 1 wherein the pulses have a length lower than 1 microsecond.

4. Electric pulse applicator according to claim 1 wherein the pulses has a length comprised between 1 and 10 nanoseconds.

5. Electric pulse applicator according to claim 1 wherein said electrically insulating coating is an insulating inorganic film.

6. Electric pulse applicator according to claim 1 wherein said electrically insulating coating is an insulating mineral film such as glass, oxide, nitride, etc...film.

7. Electric pulse applicator according to claim 1 wherein said electrically insulating coating is an insulating organic film such as an insulating cellulose film or an insulating lipidic film.

8. Electrode intended to be introduced into and/or at the vicinity of a conductive material to be treated, for an electric pulse applicator for the treatment of conductive material, said electric pulse applicator comprising a pulse generator sending pulses to the electrodes having a slope (dE/dt) comprised between 10¹⁴ and 10¹⁸ V/m/s, wherein said electrode includes a conductive main body and an electrically insulating coating.

9. Electrode according to claim 8 wherein said electrically insulating coating is an insulating inorganic film.

10. Electrode according to claim 8 wherein said electrically insulating coating is an insulating mineral film such as glass, oxide, nitride, etc...film.

11. Electrode according to claim 8 wherein said electrically insulating coating is an insulating organic film such as an insulating cellulose film or an insulating lipidic film.

12. Method for applying an electric field into a conductive material in such a way as to modify its properties wherein it comprises at least the following steps of:
- positioning at least one electrode comprising a conductive main body and an electrically insulating coating into and/or at the vicinity of the conductive material to be treated,
- sending pulses to the electrode having a slope of the raising front (dE/dt) comprised between 10¹⁴ and 10¹⁸ V/m/s.

13. Method according to claim 12 wherein the pulses have an amplitude of about 10 to 200 kV/cm and a pulse length of one or several tens or hundreds of picoseconds to one or several tens or hundreds of nanoseconds.

14. Method according to claim 12 wherein the pulses have a length lower than 1 microsecond.

15. Method according to claim 12 wherein the pulses have a length comprised between 1 and 10 nanoseconds.
